# EUROPEAN PATENT APPLICATION

(11) **EP 4 786 486 A1**
(43) Date of publication of application: **05.08.2026**
(21) Application number: 24871115.2
(22) Date of filing: 25.09.2024
(51) Int. Cl.: C07K 7/06, C07K 14/47, A61P 9/06, C12N 15/09

(54) **PEPTIDES FOR TREATING CARDIAC DISEASES**

(30) Priority: 25.09.2023 ES 202330798
(71) Applicant: Universidad Complutense De Madrid, 28040 Madrid (ES); Consejo Superior de Investigaciones Científicas, 28006 Madrid (ES); Centro Nacional de Investigaciones Cardiovasculares Carlos III (F.S.P.), 28029 Madrid (ES)
(72) Inventor: DELPÓN MOSQUERA, Eva, 28003 Madrid (ES); CABALLERO COLLADO, Ricardo, 28924 Alcorcón (ES); CRESPO GARCÍA, Teresa, 28040 Madrid (ES); CÁMARA CHECA, Anabel, 28040 Madrid (ES); RAPÚN JIMÉNEZ, Josu, 28040 Madrid (ES); TAMARGO MENÉNDEZ, Juan, 28003 Madrid (ES); GONZÁLEZ MUÑIZ, María del Rosario, 28006 Madrid (ES); NÚÑEZ VILLANUEVA, Diego, 28006 Madrid (ES); PÉREZ DE VEGA, Mª Jesús, 28006 Madrid (ES); CUADROS HIGUERAS, Carlos, 28006 Madrid (ES); ESTEBAN ARDERIUS, Pablo, 28006 Madrid (ES); FILGUEIRAS RAMA, David, 28029 Madrid (ES); JALIFE SACAL, José, 28029 Madrid (ES)
(74) Representative: Pons IP
(86) International application number: PCT/ES2024/070586
(87) International publication number: WO 2025/068623

(57) **Abstract**

The present invention relates to peptides with 11-12 amino acids for use thereof as a medicine in cardiac diseases, especially ventricular arrhythmias in patients with cardiac hypertrophy, cardiac insufficiency (CI) and/or hereditary loss-of-function syndromes in the Navi.5 or Kir2.1 channels. The peptide with 11 amino acids and the synthetic analogues with 11-12 amino acids include a nuclear localisation signal, activate *KCNJ2* transcription, prevent the decrease in the density of I_{Na} and I_{K1} caused by Cl, and significantly increase the density of I_{Na} and I_{K1} in cases of Cl. The invention also includes cDNA molecules that encode these peptides, the vectors and/or the cells containing them and pharmaceutical compositions containing same for use thereof in the prevention and/ or treatment of cardiac diseases.

## Description

### TECHNICAL SECTOR

The present invention belongs to the field of biotechnology and peptide synthesis with application in the pharmaceutical sector. More specifically, it relates to peptides with ionic current modulating properties in cardiomyocytes.

### BACKGROUND OF THE INVENTION

Cardiac insufficiency (CI) is a very prevalent syndrome characterised by high morbidity and mortality (around 50% in the first 5 years). Ventricular arrhythmias are heart rhythm disturbances that can trigger ventricular fibrillation and sudden cardiac death (SCD). SCD of arrhythmic origin is responsible for up to 50% of deaths in patients with CI. In patients with CI and life-threatening ventricular arrhythmias, the implantation of a defibrillator or resynchroniser has been shown to reduce the incidence of SCD due to ventricular arrhythmias and ventricular fibrillation. However, these are very expensive devices, which cause significant adverse effects (risk of infections, inappropriate discharges, mortality increases with the number of crashes, etc.) that limit patients' quality of life.

In patients with CI, there is a change in the electrical properties of cardiomyocytes which consists of a reduction in both Na and inwardly rectifying K currents (I_{Na} and I_{K1}) as a result of a decrease in the number of Nav1.5 and Kir2.1 channels, said channels generating these currents, respectively. These changes brought about by CI are called "electrical remodelling". Electrical remodelling facilitates the occurrence of life-threatening ventricular arrhythmias.

Currently approved and available antiarrhythmic drugs (AADs) for therapeutic use produce proarrhythmic effects, which are more pronounced the higher the patient's arrhythmic risk. Therefore, in patients with Cl, electrical, anatomical and histological remodelling facilitate the occurrence of arrhythmias, reduces the effect of AADs and increases sensitivity to their proarrhythmic actions.

The AADs of group I (hydroxyquinidine, procainamide, disopyramide, lidocaine, propafenone, and flecainide) inhibit I_{Na} by blocking the Nav1.5 channels and, as a result, can lead to dangerous proarrhythmic effects. In fact, they are contraindicated in patients with CI because they greatly reduce cardiac excitability in patients who already have compromised cardiac excitability as a result of electrical remodelling. To that end, they increase mortality in patients with Cl and arrhythmias.

The AADs of group II (β-adrenergic receptor antagonists or beta-blockers) antagonise the proarrhythmic effects of sympathetic nervous system activation and, except in patients in whom they are contraindicated (e.g., asthmatics), they are used for the treatment of Cl. Although they are considered part of the optimal treatment of CI according to therapeutic guidelines, the mortality of patients with Cl (>50% in the first 5 years after diagnosis) due to arrhythmic causes (>50% of total mortality) remains very high.

The AADs of group III (amiodarone, dronedarone, sotalol) prolong ventricular repolarisation. Dronedarone is contraindicated in patients with Cl and sotalol is not used (unless the patient has a defibrillator) because it excessively prolongs repolarisation (the QT interval of the electrocardiogram) inducing the occurrence of ventricular arrhythmias in patients who, as a result of the electrical remodelling, already have very prolonged QT. Only amiodarone is indicated, but it causes numerous serious adverse reactions that require withdrawal within 2 years and increase mortality from non-cardiac causes.

The AADs of group IV (verapamil and diltiazem) block calcium channels and are contraindicated in patients with CI because they decrease contractility.

The AADs of groups **I,** III and IV act by inhibiting cardiac ionic currents by blocking the channels that generate them. In various cardiac pathologies, it would be necessary to be able to increase I_{Na} and/or I_{K1} in human cardiomyocytes. This need is particularly imperative in patients with Cl, but it is not limited to them. The reduction in I_{Na} decreases myocardial excitability, slowing the conduction of the cardiac electrical impulse, which facilitates the occurrence of arrhythmias. The reduction in I_{K1}, in addition to reducing excitability, prolongs cardiac repolarisation, which prolongs the QT interval of the electrocardiogram, being an additional factor that also promotes the occurrence of arrhythmias.

However, at the present time, no specific pharmacological tools capable of increasing either I_{K1} or I_{Na} are available. From the point of view of pharmacological treatment of life-threatening ventricular arrhythmias, patients with CI are therapeutic orphans.

Two compounds used in the treatment of type 2 diabetes (dapagliflozin and empagliflozin) have been reported to reduce morbidity and mortality in patients with CI and are furthermore suspected to have an antiarrhythmic effect (Braunwald E. Gliflozins in the Management of Cardiovascular Disease. N Engl J Med. 2022;386:2024-2034). On the other hand, incubation of healthy human cardiomyocytes with these compounds has been demonstrated to increase the current of Na+ and K+ (Dago, M. et al. Empagliflozin and Dapagliflozin Increase Na+ and Inward Rectifier K+ Current Densities in Human Cardiomyocytes Derived from Induced Pluripotent Stem Cells (hiPSC-CMs) Cells 2022; 11:3707). The authors propose that the increase in I_{Na} and I_{K1} might contribute to the cardioprotective effect.

### DESCRIPTION OF THE INVENTION

Peptides for treating cardiac diseases.

Given the need to find a new therapeutic approach to treat cardiac diseases such as ventricular arrhythmias in patients with CI, among others, a decision was made to further study the Nav1.5 cardiac sodium channel, encoded by the gene *SCN5A*. Analysis of the protein made it possible to identify a nuclear localisation signal (NLS) at the N-terminus of Nav1.5 (NCBI protein sequence reference number: NP_000326.2). Said signal is located between amino acids 13 and 37 of Nav1.5 (SEQ ID NO: 1) and is contained in a 132-amino acid peptide, described by Matamoros, M. et al. (Nav1.5 N-terminal domain binding to α1-syntrophin increases membrane density of human Kir2.1, Kir2.2 and Nav1.5 channels. Cardiovasc Res. 2016;110:279-290). This 132-amino acid peptide also contains an α1-syntrophin binding site and exerts a chaperone effect by increasing the density of the Nav1.5, Kir2.1 and Kir2.2 channels in the cytoplasmic membrane.

With the aim of modulating the expression of *SCN5A* and the gene that encodes Kir2.1 (*KCNJ2*), peptides have been designed that maintain the NLS but are smaller in size to facilitate access to the cell nucleus. Specifically, they are peptides with 11-12 amino acids located between positions 16 and 25 or 26 (SEQ ID NO: 2), both included, of Nav1.5. This amino acid sequence is R¹-X₁X₂RESLAX₃X₄X₅-R², where R¹ = R, hydrogen (H), acetyl (Ac), palmitoyl (Pal), Ac-R or Pal-R, X₁= F, Y or W; X₂= T, S, C or M; X₃= A, G, V, L or I; X₄= I, G, A, V or L and X₅= E or D and R² = OH, NH₂, K-NH2, K(SF)-NH₂, or KR-NH₂, where SF refers to a fluorescent probe. Preferably, the sequence is selected from the group consisting of: SEQ ID NO: 3 (referred to internally and also in this specification as DECA-11), Pal-SEQ:ID NO: 23-NH₂ and/or Pal-SEQ ID NO: 25-NH₂.

When analysing the effect of SEQ ID NO: 2, and more specifically SEQ ID NO: 3, on the *SCN5A* promoter and the consequent transcription of the gene, no significant effect could be observed. However, the 11-amino acid peptide is capable of activating transcription of *KCNJ2* by exerting a protranscriptional effect over the human minimal promoter of the *KCNJ2* gene. This surprising result encouraged the analysis of the effects of the peptide SEQ ID NO: 2, and more specifically SEQ ID NO: 3, over I_{Na} and I_{K1} in a mouse model with Cl, obtaining positive results.

Another aspect of the invention relates to SEQ ID NO: 2, preferably SEQ ID NO: 3, Pal-SEQ:ID NO: 23-NH₂ and Pal-SEQ ID NO: 25-NH₂, for use thereof as a medicine, more specifically for use thereof as a medicine in cardiac diseases, which include: ventricular arrhythmias associated with cardiac hypertrophy, cardiac insufficiency and hereditary syndromes secondary to mutations in the *SCN5A* or *KCNJ2* genes resulting in the loss of function in the Nav1.5 or Kir2.1 channels, such as, for example, Brugada Syndrome, Progressive cardiac conduction defect syndrome or Andersen-Tawil syndrome, among others.

A third aspect of the invention relates to a DNA molecule that encodes the 11-amino acid peptide, in other words, cDNA molecules that encode the peptides of the variants of SEQ ID NO.: 2 (SEQ ID NO: 5-19), and preferably the cDNA molecules that encode DECA-11 (SEQ ID NO: 5), Pal-SEQ:ID NO: 23-NH₂ and Pal-SEQ ID NO: 25-NH₂ (SEQ ID NO:27 and SEQ ID NO:28).

Another aspect of the invention relates to SEQ ID NO: 5-19, 27-28, preferably SEQ ID NO: 5, SEQ ID NO:27 and SEQ ID NO:28 for use thereof as a medicine, more specifically for use thereof as a medicine in cardiac diseases, which include: ventricular arrhythmias associated with cardiac hypertrophy, cardiac insufficiency and hereditary syndromes secondary to mutations in the *SCN5A* or *KCNJ2* genes resulting in the loss of function in the Nav1.5 or Klr2.1 channels, such as, for example, Brugada Syndrome, Progressive cardiac conduction defect syndrome or Andersen-Tawil syndrome, among others.

The invention also relates to vectors including any of these cDNA molecules (SEQ ID NO: 5-19, 27-28) and eukaryotic or prokaryotic cells containing these vectors, or any of the cDNA sequences (SEQ ID NO: 5-19, 27-28), or any of the peptide sequences derived from SEQ ID NO: 2, preferably SEQ ID NO: 3, Pal-SEQ:ID NO: 23-NH₂ and Pal-SEQ ID NO: 25-NH₂.

A further aspect of the invention relates to a pharmaceutical composition comprising a peptide of sequence SEQ ID NO: 2, preferably SEQ ID NO: 3, Pal-SEQ:ID NO: 23-NH₂ and Pal-SEQ ID NO: 25-NH₂, and a pharmaceutically acceptable excipient.

Likewise, the invention also relates to a pharmaceutical composition comprising any of the cDNA molecules defined by SEQ ID NO: 5-19, 27-28 and a pharmaceutically acceptable excipient.

Another aspect of the invention relates to pharmaceutical compositions including vectors as described above, in other words, comprising any of these cDNA molecules (SEQ ID NO: 5-19; 27-28) and a pharmaceutically acceptable excipient. It also relates to pharmaceutical compositions including eukaryotic or prokaryotic cells containing these vectors, or any of the cDNA sequences (SEQ ID NO: 5-19, 27-28), or any of the peptide sequences derived from SEQ ID NO: 2, preferably SEQ ID NO: 3, Pal-SEQ:ID NO: 23-NH₂ and Pal-SEQ ID NO: 25-NH₂, and a pharmaceutically acceptable excipient.

Another aspect of the invention relates to any of the pharmaceutical compositions described in this specification for use thereof in the prevention and/or treatment of cardiac diseases, which include: ventricular arrhythmias associated with cardiac hypertrophy, cardiac insufficiency and hereditary syndromes secondary to mutations in the *SCN5A* or *KCNJ2* genes resulting in the loss of function in the Nav1.5 or Kir2.1 channels, such as, for example, Brugada Syndrome, Progressive cardiac conduction defect syndrome or Andersen-Tawil syndrome, among others.

**Table 1. Sequence table**

| | |
|---|---|
| SEQ ID NO: 1 | FRRFTRESLAAIEKRMAEKQARGST |
| SEQ ID NO: 2 | XXRESLAXXX, which corresponds to R¹-X₁X₂RESLAX₃X₄X₅-R² in this specification |
| SEQ ID NO: 3 | RFTRESLAAIE |
| SEQ ID NO: 4 | EKRMAEKQARGSTTL |
| SEQ ID NO: 5 | AGGTTCACACGGGAGTCCCTGGCAGCCATCGAG |
| SEQ ID NO: 6 | AGGTATACACGGGAGTCCCTGGCAGCCATCGAG |
| SEQ ID NO: 7 | AGGTGGACACGGGAGTCCCTGGCAGCCATCGAG |
| SEQ ID NO: 8 | AGGTTCTCTCGGGAGTCCCTGGCAGCCATCGAG |
| SEQ ID NO: 9 | AGGTTCTGTCGGGAGTCCCTGGCAGCCATCGAG |
| SEQ ID NO: 10 | AGGTTCATGCGGGAGTCCCTGGCAGCCATCGAG |
| SEQ ID NO: 11 | AGGTTCACACGGGAGTCCCTGGCAGGTATCGAG |
| SEQ ID NO: 12 | AGGTTCACACGGGAGTCCCTGGCAGTTATCGAG |
| SEQ ID NO: 13 | AGGTTCACACGGGAGTCCCTGGCATTAATCGAG |
| SEQ ID NO: 14 | AGGTTCACACGGGAGTCCCTGGCAATTATCGAG |
| SEQ ID NO: 15 | AGGTTCACACGGGAGTCCCTGGCAGCCGGTGAG |
| SEQ ID NO: 16 | AGGTTCACACGGGAGTCCCTGGCAGCCGCTGAG |
| SEQ ID NO: 17 | AGGTTCACACGGGAGTCCCTGGCAGCCGTTGAG |
| SEQ ID NO: 18 | AGGTTCACACGGGAGTCCCTGGCAGCCTTAGAG |
| SEQ ID NO: 19 | AGGTTCACACGGGAGTCCCTGGCAGCCATCGAT |
| SEQ ID NO: 20 | GAGAAGCGCATGGCAGAGAAGCAAGCCCGCGGC TCAACCACCTTG |
| SEQ ID NO: 21 | cDNA that encodes the human Nav1.5 channels (see sequence listing) |
| SEQ ID NO: 22 | cDNA that encodes the human Klr2.1 channels (see sequence listing) |
| Ac-SEQ ID NO: 23(SF)-NH₂ | Ac-RFTRESLAAlEK(SF)-NH₂ |
| Pal-SEQ ID NO: 23(SF)-NH₂ | Pal-RFTRESLAAIEK(SF)-NH₂ |
| Pal-SEQ ID NO: 23-NH₂ | Pal-RFTRESLAAIEK-NH₂ |
| Ac-SEQ ID NO: 3-NH₂ | Ac-RFTRESLAAIE-NH₂ |
| Pal-SEQ ID NO: 3-NH₂ | Pal-RFTRESLAAIE-NH₂ |
| Ac-SEQ ID NO: 25-NH₂ | Ac-FTRESLAAIEK-NH₂ |
| Pal-SEQ ID NO: 25-NH₂ | Pal-FTRESLAAIEK-NH₂ |
| Ac-SEQ ID NO: 26-NH₂ | Ac-FTRESLAAIEKR-NH₂ |
| SEQ ID NO: 27 | AGGTTCACACGGGAGTCCCTGGCAGCCATCGAG AAG |
| SEQ ID NO: 28 | TTCACACGGGAGTCCCTGGCAGCCATCGAGAAG |

### BRIEF DESCRIPTION OF THE FIGURES

To complement the description that is being made and for the purpose of helping to better understand the features of the invention, a set of figures is included as an integral part of said description, wherein the following has been depicted in an illustrative and non-limiting manner:
**Figure 1****.** Density of the current generated by the human Nav1.5 channels as a function of the membrane potential of the test pulse in the presence or absence of two peptides of different sizes (15-25 that is DECA-11 (SEQ ID NO: 3) or 25-39 (SEQ ID NO: 4)) which form part of the N-terminus of the Nav1.5 channel. In each panel, the peptide studied is named describing the position of the first and last amino acid of the sequence of the human Nav1.5 channel.
**Figure 2****.** Density of the current generated by the human Kir2.1 channels as a function of the membrane potential of the test pulse in the presence or absence of the peptide 15-25 that is DECA-11 (SEQ ID NO: 3).
**Figure 3****.** I_{Na} (Fig. 3A) and I_{K1} (Fig. 3B) density relationships as a function of the membrane potential of the applied pulse that are recorded in human cardiomyocytes derived from induced pluripotent stem cells.
**Figure 4****.** Traces of sustained or late I_{Na} (I_{NaL}) that are recorded in human cardiomyocytes derived from induced pluripotent stem cells in the presence (Fig. 4B) or absence (control) of DECA-11 (Fig. 4A). I_{NaL} was considered the sustained current sensitive to tetrodotoxin TTX (10 µM) and quantified as a percentage of the current peak (Fig. 4C).
**Figure 5****.** Luciferase assays to analyse the effect of DECA-11 (SEQ ID NO: 3) on the transcription of *SCN5A* (Fig. 5A) and *KCNJ2* (Fig. 5B). Relative luminescence is expressed in arbitrary units (AU).
**Figure 6****.** Scheme of the protocol followed for the generation of the murine model with HFrEF. Time is expressed in weeks (S). The weeks in which baseline (ECO1) and final (ECO2) echocardiograms were performed before transfer to our laboratory are indicated. Animals that underwent surgery without aortic ligation are referred to as Sham and those that had their aorta ligated are referred to as TAC.
**Figure 7****.** Capacitance (pF) of dissociated cardiomyocytes from Sham and TAC mice treated with Aav-dt-T (control) or Aav-DECA-11 (Aav-SEQ ID NO: 5).

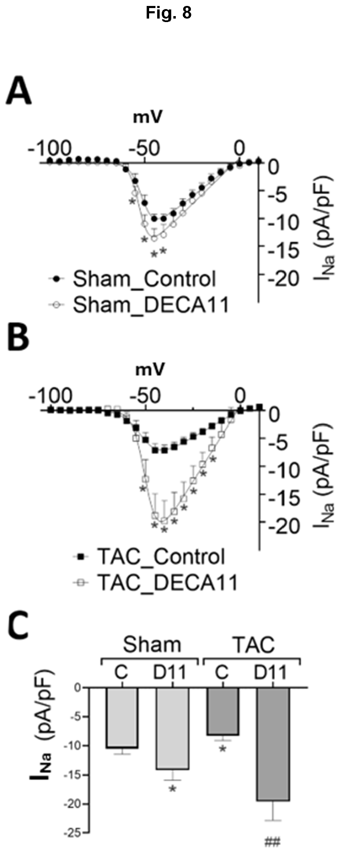

**Figure 8****.** I_{Na}-voltage density relationships obtained in dissociated cardiomyocytes from Sham mice treated or not with DECA-11 (A) and TAC mice treated or not with DECA-11 (B); summary of results (C). In panel C, C stands for Control and D11 stands for DECA-11.
**Figure 9****.** I_{K1}-voltage density relationships obtained in dissociated cardiomyocytes from Sham mice treated or not with DECA-11 (A) and TAC mice treated or not with DECA-11 (B); summary of results (C). In panel C, C stands for Control and D11 stands for DECA-11.
**Figure 10****.** Images of HEK293 cells treated for 24 hours with Ac-SEQ ID NO: 23(SF)-NH₂ and Pal-SEQ ID NO: 23(SF)-NH₂ at the concentration of 10 µM obtained by fluorescence microscopy.
**Figure 11****.** Conformational analysis by circular dichroism of Ac-SEQ ID NO: 3-NH₂, Ac-SEQ ID NO: 25-NH₂ and Ac-SEQ ID NO: 26-NH₂, in H₂O and mixtures of H₂O/TFE (60:40), carried out at 5°C and with scans between 190 and 250 nm.
**Figure 12****.** Stability graph of Ac-SEQ ID NO: 26-NH₂ in human blood serum/H₂O (50:50), with incubation at 37°C and control by UPLC-MS at 24 and 72 hours.

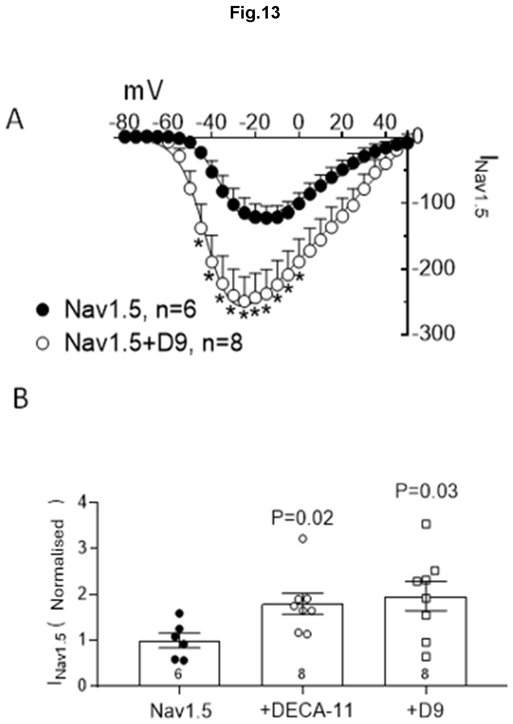

**Figure 13****.** I_{Na}-voltage density relationships obtained in CHO cells expressing human Nav1.5 channels treated or not with the peptide Pal-SEQ ID NO: 23-NH₂ (indicated by D9 in this figure), at a concentration of 10 µM (A). Panel B shows the normalised density of the maximum peak of I_{Na} recorded in CHO cells expressing Nav1.5 channels that had been treated (white squares) or not (black circles) for 24 hours with Pal-SEQ ID NO: 23-NH₂, at a concentration of 10 µM. The white circles represent the normalised density of the maximum peak of I_{Na} recorded in CHO cells expressing Nav1.5 channels together with the cDNA that encodes the fragment containing amino acids 15-25 (DECA-11) described in SEQ ID NO: 3 (SEQ ID NO: 5).

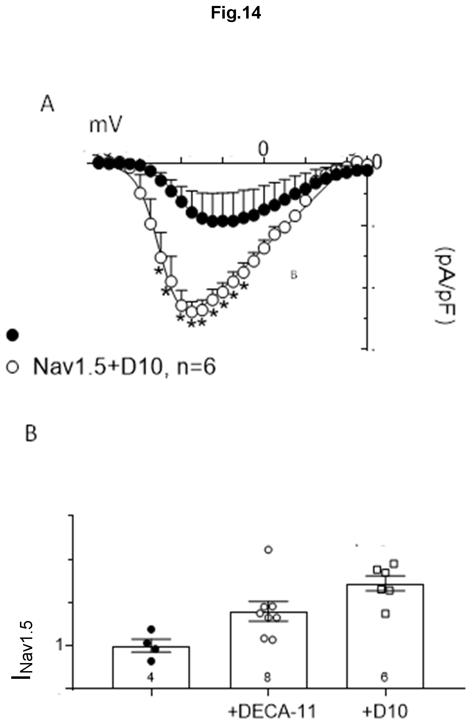

**Figure 14****.** I_{Na}-voltage density relationships obtained in CHO cells expressing human Nav1.5 channels treated or not with the peptide Pal-SEQ ID NO: 25-NH₂ (D10 in this figure), at a concentration of 10 µM (A). Panel B shows the normalised density of the maximum peak of I_{Na} recorded in CHO cells expressing Nav1.5 channels that had been treated (white squares) or not (black circles) for 24 hours with Pal-SEQ ID NO: 25-NH₂, at a concentration of 10 µM. The white circles represent the normalised density of the maximum peak of I_{Na} recorded in CHO cells expressing Nav1.5 channels together with the cDNA that encodes the fragment containing amino acids 15-25 (DECA-11) described in SEQ ID NO: 3 (SEQ ID NO: 5).

### PREFERRED EMBODIMENT OF THE INVENTION

The present invention is illustrated by the following examples, which are not intended to be limiting in scope.

### Example 1. Peptide design

Among other tools, in the analysis of Nav1.5 (NCBI: NP_000326.2), a nuclear localisation site prediction algorithm was used (Nguyen Ba AN, et al. NLStradamus: a simple Hidden Markov Model for nuclear localization signal prediction. BMC Bioinformatics. 2009; 10:202) with which an NLS was identified (SEQ ID NO: 1) between amino acids 13 and 37, both included, of Nav1.5. While the size of the NLS is not excessive, smaller peptides were designed to identify a minimal fragment capable of accessing the cell nucleus and increasing I_{Na} and I_{K1}. Among them, an 11-amino acid peptide (SEQ ID NO: 3) and another 15-amino acid peptide (SEQ ID NO: 4), which were generated in the laboratory, were selected. The 11-amino acid peptide was selected with a view to leaving an Arginine (R) residue at the N-terminus to facilitate the deletion of the peptide if necessary to increase its biological stability.Sequences SEQ ID NO: 23-26 and their modifications were designed to study the ability to cross the cytoplasmic membrane, and/or preferred conformation, and/or stability in blood serum, and/or biological activity.

### Example 2. Analysis of density of current generated

In addition to their ability to enter the nucleus of cells, there was a search for peptides capable of increasing the density of current generated by the human Nav1.5 and Kir2.1 channels, and therefore both aspects were analysed.

### Example 2.1. Density of current generated by the human Nav1.5 channels

To analyse the effect of the peptides designed in example 1 on I_{Na} and I_{K1}, the cDNA that encodes SEQ ID NO: 3 or SEQ ID NO: 4, described in SEQ ID NO: 5 and 20, respectively, was cloned into a pcDNA3.1^{(+)®} vector (Invitrogen) for transfection of Chinese hamster ovary (CHO) cells that were in turn transfected with the cDNA that encodes the human Nav1.5 channels (SEQ ID NO: 21). The CHO cells were further co-transfected with the EBO-pcD leu 2 vector (0.5 µg), which encodes the expression of the CD8 surface antigen. The subsequent addition of anti-CD8 antibody, bonded to Dynabeads M450^{®} microspheres, allowed the cells that had been transfected to be identified. After 48 hours, an aliquot of the transfected cell suspension was placed in the chamber mounted on the stage of an inverted microscope (TMS, Nikon) and perfused at the rate of 1 ml/min with external solution (in mM): 50 NaCI, 80 CsCl, 1 CaCl₂, 1.5 MgCl₂, 5 HEPES and 5 glucose (pH 7.35 with CsOH). The micropipettes were obtained from borosilicate capillaries (mod. GD-1, Narishige) using a programmable horizontal puller (Sutter mod. P-2000) and polishing its mouth by heat in a microforge (mod. MF-83, Narishige). The micropipettes (tip resistance <1.5 MΩ) were filled with an internal solution containing (mM): 10 NaF, 110 CsF, 20 CsCI, 10 HEPES and 10 EGTA (pH 7.35 with CsOH). The micropipettes were brought closer to the cell membrane using a Narishige MO-103 micromanipulator and a small amount of suction was applied, the membrane portion included in the pipette lumen was invaginated, forming a high-resistance seal [of the order of GΩ, with a cell-attached configuration]. After the formation of the seal, the patch membrane was broken by a small additional suction, which enabled the recording of the current using the whole-cell configuration of the membrane patch-clamp technique. The currents were recorded at 20-22°C using an Axopatch 200B amplifier, filtered at half the sampling frequency and stored in a computer for further analysis. Data acquisition, analysis of said data and pulse protocols were controlled by the pCLAMP software. The capacitive current artefacts generated after the application of pulses from 0 mV to +10 mV were recorded at 50 kHz (filtered at 10 kHz) to calculate the capacitance of the cell, the access resistance and the input impedance. The sampling frequency was 50 kHz and the data, filtered at a frequency of 25 kHz, were stored on hard disks for further analysis using the CLAMPFIT application of the pCLAMP software. I_{Na} was recorded by applying 50 ms pulses from a holding potential of -120 mV to potentials between -80 and +50 mV in 5 mV increments. The amplitude of the peak of I_{Na} was normalised to the capacitance of each cell, thus calculating the density of I_{Na} (pA/pF). The I_{Na} -voltage density curves were obtained by plotting the density of I_{Na} as a function of the membrane potential of the applied pulse.

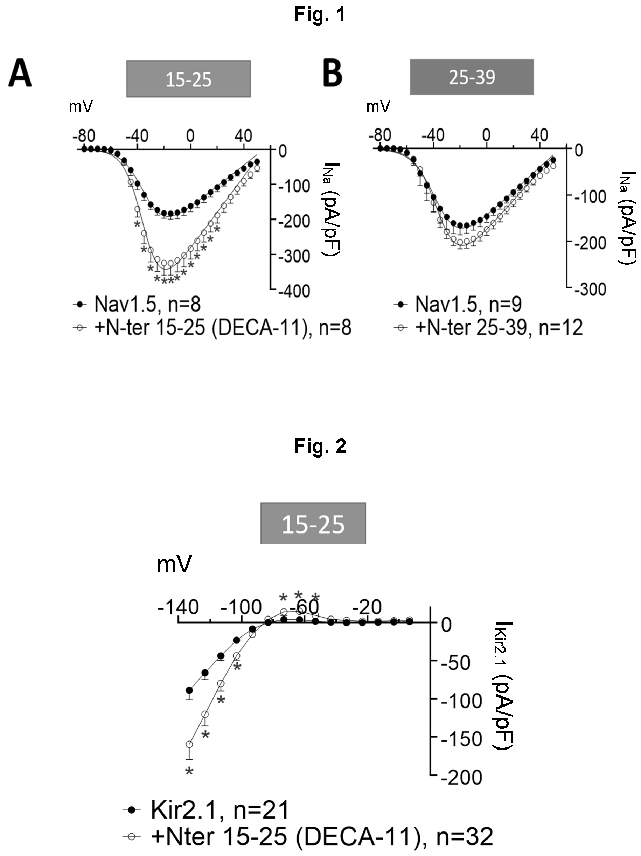

Figure 1 plots the I_{Na} -voltage density curves in cells expressing the Nav1.5 channels (black symbols) and in cells expressing the Nav1.5 channels together with the cDNA that encodes the fragment containing amino acids 15-25 described in SEQ ID NO: 3 (SEQ ID NO: 5) or the one that encodes amino acids 25-39, described in SEQ ID NO: 4, (SEQ ID NO: 20) (white symbols). As can be observed, the fragment containing amino acids 15-25 (SEQ ID NO: 3) was able to significantly increase the density of I_{Na} at various membrane potentials. On the contrary, the fragment containing amino acids 25-39 (SEQ ID NO: 4) did not. Each point represents the mean±SEM of "n" experiments obtained from at least 3 different CHO cell plates. One-way ANOVA followed by the Tukey test. * P<0.05 vs. cells transfected only with Nav1.5 channels.

### Example 2.2. Density of current generated by the human Kir2.1 channels.

In view of the results of example 2.1, in this example only the peptide described in SEQ ID NO: 3 was analysed using its cDNA (SEQ ID NO: 5).

To do this, CHO cells were transfected with the cDNA that encodes the human cardiac channels (Kir2.1) that generate I_{K1} (Origene, USA) (SEQ ID NO: 22), co-transfecting cells, or not, with the cDNA described in SEQ ID NO: 5. In these experiments, the external solution that bathed the cells and the internal solution that filled the micropipettes had the following composition (mM): 136 NaCl, 4 KOI, 1.8 CaCl₂, 1 MgCl₂, 10 HEPES and 10 glucose (pH=7.4 with NaOH) and 80 K-aspartate, 50 KOI, 10 KH2PO₄, 3 MgATP, 10 HEPES, 5 EGTA (pH=7.25 with KOH), respectively. The sampling frequency was 4 kHz and the data, filtered at a frequency of 2 kHz, were stored on hard disks for further analysis using the CLAMPFIT application of the pCLAMP software. The current (I_{Kir2.1}) was recorded by applying 250 ms pulses from -120 to +20 mV from a holding potential of -60 mV. The density of the same was obtained by normalising the amplitude of I_{Kir2.1} at the end of the pulse by the capacitance of each cell (pA/pF). Figure 2 shows the density of I_{Kir2.1} as a function of the membrane potential (mV) of the applied pulse in cells transfected with the cDNA that encodes the Kir2.1 channels and co-transfected or not with the cDNA that encodes the peptide SEQ ID NO: 3. Like in example 2.1, each point represents the mean±SEM of "n" experiments obtained from at least 3 different CHO cell plates. One-way ANOVA followed by the Tukey test. * P<0.05 vs. cells transfected only with Kir2.1 channels. Again, SEQ ID NO: 3 was able to significantly increase the density of I_{Kir2.1}.

### Example 2.3. Density of current generated in human cardiomyocytes

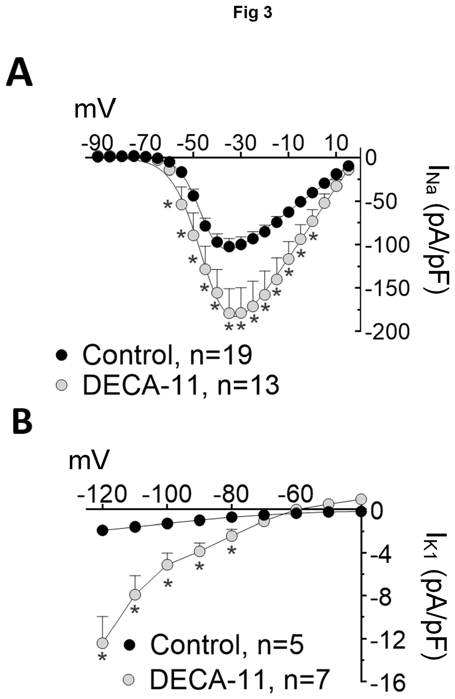

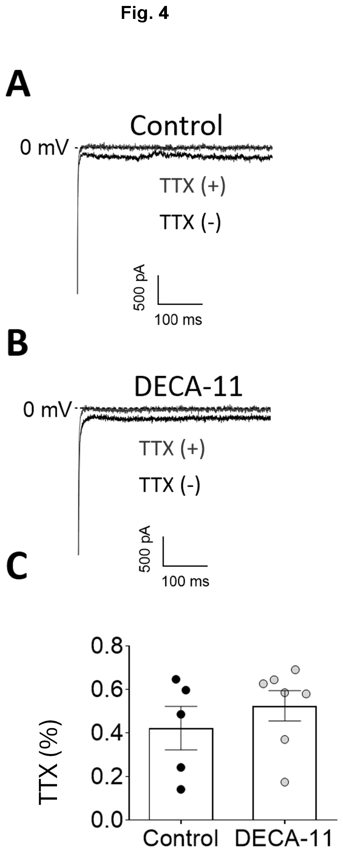

Human-induced pluripotent stem cell-derived cardiomyocytes (hiPSC-CM) were infected with adeno-associated virus serotype 9 Ad-td-Tomato (control) or with an adeno-associated virus serotype 9 containing the cDNA of the peptide 15-25 (SEQ ID NO: 5) and that of td-Tomato (Aav-DECA-11; Aav-SEQ ID NO: 5). Commercial hiPSC-CM (iCell Cardiomyocytes^{®}) were used and thawed and maintained in culture for 10 days following the protocols described by the manufacturer (Cellular Dynamics, USA). Infection with Ad-td-Tomato (a red fluorescent protein) or Ad-DECA-11 was carried out 48 hours prior to recording currents using the patch-clamp technique in the whole-cell configuration. The external solution that bathed the cardiomyocytes and the internal solution that filled the micropipettes had the following composition when I_{Na} was recorded (mM): 20 NaCI, 1.5 MgCl₂, 1 CaCl₂, 115 CsCl, 5 HEPES, 10 glucose and nifedipine (1 µM) (pH= 7.35 with CsOH) and 10 NaF, 110 CsF, 10 EGTA, 20 CsCI and 10 HEPES (pH=7.35 with CsOH), respectively. To record I_{K1}, the composition of the external solution was (mM): 148 NaCI, 1 MgCl₂, 1.8 CaCI₂, 5.4 KOI, 0.4 NaH₂PO₄, 15 HEPES, 11 glucose and nifedipine (5 µM) (pH= 7.35 with NaOH) and that of the internal solution was (mM): 148 KOI, 1 MgCI₂, 5 EGTA, 2 creatine, 5 Mg-ATP, 5 phosphocreatine, 5 HEPES (pH=7.2 with KOH). Figure 3 shows that infection with the virus that encodes DECA-11 is able to significantly increase the density of I_{Na} and I_{K1} recorded in human iPSC-derived cardiomyocytes. In this figure, each point represents the mean±SEM of "n" experiments/cardiomyocytes obtained from at least 3 different culture plates. * P<0.05 versus Ad-td-Tomato infected cardiomyocytes. One-way ANOVA followed by the Tukey test. In patients with CI, the sustained component of I_{Na} called I_{NaL} increases. This increase leads to a rise in intracellular concentrations of Na⁺, which, in turn, alters intracellular Ca²⁺ handling and prolongs the duration of action potentials leading to proarrhythmic effects. To that end, there was a desire to see if DECA-11 modified I_{NaL}. To do this, 500 ms pulses from -120 to -45 mV were applied to human iPSC-derived cardiomyocytes and I_{Na} and I_{NaL} were recorded using the "external" and "internal" solutions described above. I_{NaL} is described as the component of the sustained current that is sensitive to tetrodotoxin (TTX) (10 µM) and for quantification it was measured as a percentage of the current peak (%). Figure 4 shows that DECA-11 does not increase I_{NaL}.

All the data presented so far demonstrate that SEQ ID NO: 3 (and by extension any of the variations within SEQ ID NO.: 2) is able to increase the density of I_{Na} and I_{K1} in heterologous expression systems and in human cardiomyocytes without modifying that of I_{NaL}.

### Example 3. Protranscriptional effect of DECA-11

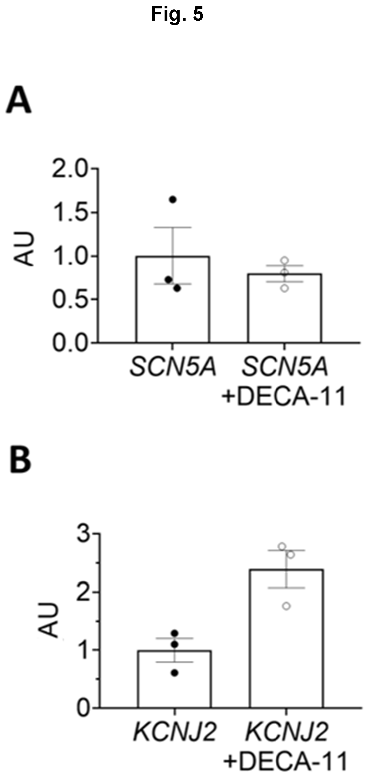

To study the protranscriptional effect, hEK-293 cells were seeded in 96-well plates and transfected with 300 ng of pLightSwitch-Prom luciferase vectors encoding the human minimal promoter (≈1000 base pairs) of the *SCN5A* or *KCNJ2* genes (Active Motif) and co-transfected or not with the vector containing the cDNA of the peptide 15-25 (SEQ ID NO: 5). 48 hours after transfection, luciferase assays were performed using the *LightSwitch Luciferase Assay* reagent, also from Active Motif, and a Berthold luminometer. All assays were performed in triplicate and each point represents the mean of each technical triplicate. It was found that the peptide described in SEQ ID NO: 3 has no significant effect on the minimal promoter of the human *SCN5A* gene (Figure 5); however, it does exert a protranscriptional effect on the minimal promoter of the human *KCNJ2* gene, which implies increased expression of the Kir2.1 channels.

### Example 4. Assays in a murine model of CI

A murine model of CI with reduced ejection fraction (HFrEF) was used to analyse the effect of SEQ ID NO: 3 on the decrease in I_{Na} and I_{K1} caused by electrical remodelling associated with Cl. The mice are subjected to transverse aortic constriction (TAC) with ligation, and pressure overload leads to the occurrence of cardiac hypertrophy and Cl. The mice were produced at the Medical Research Centre of the University of Navarra (CIMA), under the direction of Dr Aránzazu González Miqueo.

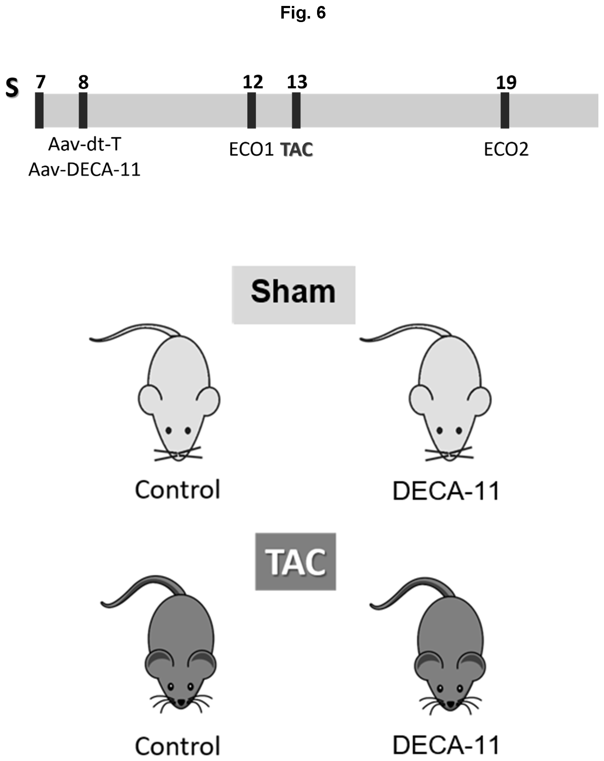

Eight-week-old mice were administered 3.5×10¹⁰ particles of adeno-associated virus type 9 that encoded either the fluorescent reporter td-Tomato (Aav-dt-T) alone, or the reporter and the peptide DECA-11. At 12 weeks of age, an echocardiographic check-up (baseline) was performed and at week 13 they underwent surgery in which the aorta was ligated (TAC animals) or not (Sham animals). The upper part of Figure 6 shows each of the procedures to which the animals were subjected according to their age (in weeks); the four groups of animals that underwent surgery with ligation (TAC) or without ligation (Sham) are shown at the bottom.

At 19 weeks of age, a new echocardiographic check-up (ECO) was performed, which showed that the TAC animals had HFrEF, as expected. That same week they were sent to the UCM animal facility. Next, the mice were heparinised and anaesthetised and then sacrificed by cervical dislocation, at a rate of one per day, and a thoracotomy was performed to remove the heart which was mounted through the aorta into a cannula connected to a Langendorff apparatus for enzymatic dissociation of the cardiac myocytes. After removal of the heart, said heart was cannulated through the aorta and placed in a Langendorff apparatus. First, the hearts were perfused with modified Tyrode's solution (composition in mM: 136 NaCI, 4 KCI, 2 MgCl₂, 1.8 CaCl₂, 10 HEPES, 10 glucose, 3 pyruvic acid and 0.1 thiamine, pH=7.4 with NaOH) bubbled with oxygen and at 37°C for 3-5 minutes. Subsequently, they were perfused for another 8 minutes with the same solution without CaCl₂. After 1-2 minutes, the heart stops beating. Next, they were perfused with Tyrode's solution without CaCl₂ supplemented with 120 u/ml collagenase type II from Worthington and 0.5 u/ml protease XIV from Sigma. Lastly, the hearts were perfused with a solution called KB of the following composition (mM): 25 KCI, 10 KH₂PO₄, 2 MgCI₂, 0.5 EGTA-K, 5 HEPES-K, 100 glutamic acid, 10 aspartic acid, 20 taurine, 5 creatine and 10 glucose (pH=7.2 with KOH). After this process has been completed, the heart was detached from the cannula and shaken in aliquots of KB solution where the myocytes were disintegrated and stored in the refrigerator for at least one hour before electrophysiological recordings were started.

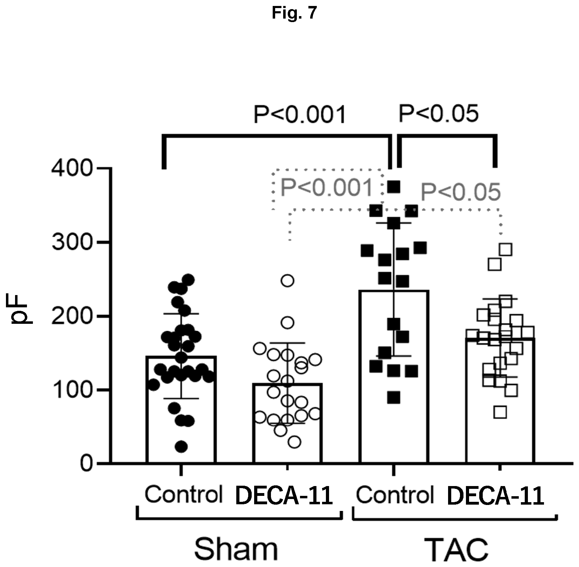

Figure 7 shows the capacitance (pF) of the dissociated cardiac myocytes from the four groups of mice (12 in total). Capacitance is an indirect and reliable measure of cell size. As can be observed, there was no significant difference in the capacitance of myocytes from Sham animals, had they been infected with Ad-td-Tomato (control) or with Aav-DECA-11. On the contrary, the capacitance of cardiomyocytes from animals subjected to the TAC procedure was significantly higher (P<0.05) than that of control Sham and DECA-11 animals. This result is in line with expectations, since the TAC animals show cardiac hypertrophy and, therefore, the size of their cardiomyocytes is larger than those of normal hearts.

The capacitance of myocytes from TAC animals treated with DECA-11 was significantly lower than that of untreated TAC animals, indicating that the administration of DECA-11 prevents the occurrence of cardiomyocyte hypertrophy associated with HFrEF.

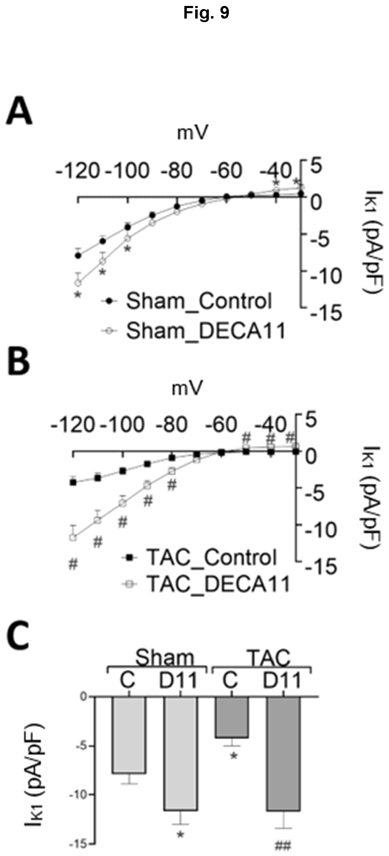

To record I_{Na} in mouse cardiomyocytes, the composition of the external solution was (mM): 4 NaCl, 1 MgCl₂, 1 CaCl₂, 0.1 CdCl₂, 133.5 CsCl, 20 HEPES and 11 glucose (pH=7.35 with CsOH) and the solution that filled the recording micropipette was (mM): 10 NaF, 110 CsF, 20 CsCl, 10 HEPES and 10 EGTA (pH 7.35 with CsOH). All other protocols and conditions were the same as those used for recording I_{Na} in human iPSC-derived cardiomyocytes (according to Example 2.3). As shown in Figure 9, in animals with HFrEF, and as a result of the electrical remodelling, the density of I_{Na} recorded in cardiomyocytes from control TAC mice was significantly lower than that of control Sham mice. Pre-treatment with DECA-11 increased the density of I_{Na} of cardiomyocytes from Sham animals and, more importantly, significantly and markedly increased the density of I_{Na} of TAC cardiomyocytes. This result demonstrates that the administration of DECA-11 prevents the decrease in the density of I_{Na} produced by HFrEF in a murine model.

As indicated above, I_{K1} was also recorded in mouse cardiomyocytes from all four groups. To do this, the composition of the external solution was (mM): 140 NaCl, 1 MgCl₂, 1 CaCl₂, 4 KCI, 10 HEPES, 2 4-aminopyridine, nifedipine (1 µM), atropine (0.1 µM), glibenclamide (10 µM) and 10 glucose, (pH= 7.35 with NaOH) and that of the internal solution was (mM): 80 K-aspartate, 50 KCI, 10 KH₂PO₄, 3 MgATP, 10 HEPES, 5 EGTA (pH=7.25 with KOH). As a result of electrical remodelling associated with HFrEF caused by transverse aortic constriction, the density of I_{K1} was significantly lower in cardiomyocytes from control TAC animals than that from control Sham animals (Figures 9A and 9B). In both Sham and TAC animals, the administration of DECA-11 significantly increases I_{K1}. In fact, the density of I_{K1} in TAC animals pretreated with DECA-11 is not different from that recorded in Sham animals. These results demonstrate that the administration of DECA-11 prevents the decrease in the density of I_{K1} produced by HFrEF in a murine model.

### Example 5. Solid-phase synthesis of DECA-11 analogues

The synthesis was carried out manually using polypropylene syringes, of variable volume depending on the amount of resin used, equipped with a porous polyethylene filter plate. In general, the resin was kept in the syringe by adding the appropriate reagents and solvents in each case so that it remained covered and solvated. The polymeric support was stirred with an orbital shaker. Excess reagents, solvents and possible by-products were removed by filtration. A TentaGel resin with Rink amide spacer loaded with 0.24 mmol/g was used. A Fmoc/^{t}Bu strategy was used to elongate the linear peptides, following the protocol described in Table 2.

**Table 2. General protocol for amino acid deprotection and coupling**

| **Step** | **Reagents** | **Operation** | **Time** |
|---|---|---|---|
| 1 | DCM / DMF / DCM / DMF | Swollen | 4 x 0.5 min |
| 2 | 20% Piperidine / DMF | Fmoc deprotection | 1 x 1 min, 3 x 10 min |
| 3 | DMF / MeOH / THF / DCM / DMF | Washed | 5 x 0.5 min |
| 4 | Fmoc-AA-OH (4 eq) / HCTU (4 eq) / DIEA (4 eq) / DMF | Coupling | 1 h |
| 5 | DMF / MeOH / THF / DCM / DMF | Washed | 5 x 0.5 min |
| 6 | Ninhydrin assay | Control | - |
| 7^{a} | Fmoc-AA-OH (4 eq) / HCTU (4 eq) / DIEA (4 eq) / DMF | Coupling | 1 h |
| 8^{a} | DMF / MeOH / THF / DCM / DMF | Washed | 5 x 0.5 min |
| 9^{a} | Ninhydrin assay | Control | - |

| | | | |
|---|---|---|---|
| ^{a} Only in cases with positive ninhydrin assay in step 6. If the assay was positive in step 9, steps 7-9 were repeated. | | | |

After peptide elongation is complete, the protocol to be followed for N-terminus acetylation is described below in Table 3.

**Table 3. General protocol for N-terminus amine acetylation.**

| **Step** | **Reagents** | **Operation** | **Time** |
|---|---|---|---|
| 1 | DCM / DMF / DCM / DMF | Swollen | 4 x 0.5 min |
| 2 | 20% Piperidine / DMF | Fmoc deprotection | 1 x 1 min, 3 x 10 min |
| 3 | DMF / MeOH / THF / DCM/DMF | Washed | 5 x 0.5 min |
| 4 | 10% Ac₂O in:DMF | Acetylation | 1 x 1 min |
| | | | 3 x 10 min |
| 5 | DMF / MeOH / THF / DCM | Washed | 4 x 0.5 min |

For *N*-palmitoylated derivatives, after peptide elongation is complete, the general protocol for palmitic acid coupling is described in Table 4.

**Table 4. General protocol for palmitic acid coupling at the N-terminus.**

| **Step** | **Reagents** | **Operation** | **Time** |
|---|---|---|---|
| 1 | DCM / DMF / DCM / DMF | Swollen | 4 x 0.5 min |
| 2 | 20% Piperidine / DMF | Fmoc deprotection | 1 x 1 min, 3 x 10 min |
| 3 | DMF / MeOH / THF / DCM / DMF | Washed | 5 x 0.5 min |
| 4 | Palmitic acid (2 eq) / HCTU (3 eq) / DIEA (3 eq) / NMP | Coupling | 1 h |
| 5 | DMF / MeOH / THF / DCM | Washed | 4 x 0.5 min |

For the cleavage of resin products, the acidolytic mixture consisting of TFA:EDT:H₂O:TIPS was added in ratios of 94:2.5:2.5:1, on the resin in the syringe (approximately 1 ml of solution per 100 mg of resin). After addition of the solution, it was left to react at room temperature for 3 hours. Subsequently, the filtrates were collected in a Falcon tube and cold ethyl ether was added to precipitate the peptide. The mixture was centrifuged for 10 min at 5000 rpm and at -15°C. The supernatant was decanted and the process was repeated two more times. The dried precipitate obtained was dissolved in water or in water/acetonitrile mixtures, and freeze-dried to obtain the final crude.

The crudes were analysed by reverse phase HPLC-MS using a SunFire C18 column (2.1mm x 50mm; 3.5 µm). A gradient of CH₃CN + 0.1% formic acid and H₂O + 0.1% formic acid was used, at a flow rate of 1 ml/min and with continuous detection at a wavelength between 230-400 nm.

The reaction crudes were purified by flash chromatography with Biotage Sfär Bio C18 reversed phase cartridges, using as solvent a gradient of CH₃CN + 0.05% in H₂O + 0.05% TFA. The collected fractions were analysed by reversed phase HPLC using an Eclipse Plus C18 column (4.6mm x 150 mm, 5 µm), a gradient of CH₃CN and H₂O + 0.05% TFA, at a flow rate of 1.5 ml/min and with detection at 220 nm wavelength. The characterisation of the synthesised peptides is shown in Table 5.

**Table 5. Characterisation of the synthesised peptides.**

| Peptide | HPLC t_{R} (min) | [M+H]⁺ Calc. (HRMS) | [M+H]⁺ Observ. (HRMS) | Yield (%) | Final purity (%) |
|---|---|---|---|---|---|
| Ac-SEQ ID NO: 23(SF)-NH₂ | 6.22^{b} | 1849.8458 | 926.3 (M+2/2) | 18 | 95 |
| Pal-SEQ ID NO: 23(SF)-NH₂ | 7.89^{b} | 2046.0649 | 2046.0712 | 18 | 96 |
| Pal-SEQ ID NO: 23-NH₂ | 7.22^{b} | 1657.0291 | 1657.0289 | 25 | 98 |
| Ac-SEQ ID NO: 3-NH₂ | 5.46^{c} | 1332.7150 | 667.6 (M+2/2) | 55 | 98 |
| Pal-SEQ ID NO: 3-NH₂ | 6.30^{d} | 1529.9341 | 765.7 (M+2/2) | 13 | 97 |
| Ac-SEQ ID NO: 25-NH₂ | 5.63^{b} | 1304.7089 | 1304.7076 | 21 | 100 |
| Pal-SEQ ID NO: 25-NH₂ | 8.01^{b} | 1500.9280 | 1500.9268 | 16 | 99 |
| Ac-SEQ ID NO: 26-NH₂ | 4.76^{b} | 1460.8100 | 1460.8095 | 29 | 99 |

| | | | | | |
|---|---|---|---|---|---|
| ^{a} Ac = acetyl, Pal = palmitoyl, SF = fluorescent probe ^{b} Gradient: 5-95% (MeCN + 0.1% formic acid/H₂O + 0.1% formic acid), 10 min ^{c} Gradient: 20-95% (MeCN + 0.1% formic acid/H₂O + 0.1% formic acid), 10 min ^{d} Gradient: 2-95% (MeCN + 0.1% formic acid/H₂O + 0.1% formic acid), 10 min | | | | | |

### Example 6. Studies on access to the cell interior of peptides Ac-SEQ ID NO.: 23(SF)-NH₂ and Pal-SEQ ID NO: 23(SF)-NH₂

In this group of experiments, the ability to access the cell interior of various DECA11-derived peptides (SEQ ID NO: 3) conjugated with the fluorescein-thiourea fluorescent probe was analysed. For this purpose, a fluorescence microscopy imaging study was performed on HEK293 cells. Thus, if and only if the peptides are able to cross the cell membrane by themselves, they will generate a fluorescent signal that can be detected by fluorescence microscopy. The cells, cultured at 37°C in Dulbecco's Modified Eagle Medium (DMEM) together with 10% foetal bovine serum, 100 U/ml penicillin and 100 µg/ml streptomycin, were incubated for 24 hours with Ac-SEQ ID NO: 23(SF)-NH₂ or Pal-SEQ ID NO: 23(SF)-NH₂ at a concentration of 10 µM. After the incubation period, the cells were seeded on polylysine-coated glass coverslips placed in 35 mm dishes and left to stand for 24 hours. Subsequently, the cells were fixed by incubation with 4% paraformaldehyde for 15 minutes. After this time has elapsed, the cells were treated with 4',6-diamidino-2-phenylindole (DAPI, 300 nM) which is a fluorescent dye that binds to adenine-thymine-rich regions in DNA and, therefore, is used to mark the nucleus of cells. Lastly, the cells were incubated with PROLONG mounting medium (4 µl) and kept at rest at 4°C overnight. The following day, the corresponding images were obtained using a fluorescence microscope model "THUNDER imager 3D Tissue".

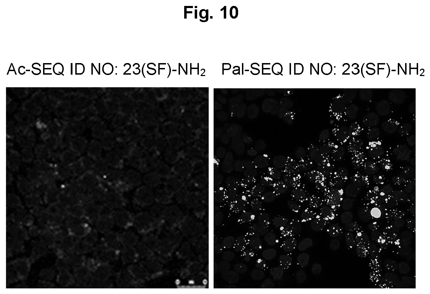

The comparative study of the permeability of the fluorescent peptides Ac-SEQ ID NO: 23(SF)-NH₂ and Pal-SEQ ID NO: 23(SF)-NH₂ is shown in Figure 10. It shows that the peptide Ac-SEQ ID NO: 23(SF)-NH₂ is not able to penetrate inside the cultured cells and therefore no green fluorescent signal is recorded. On the contrary, the peptide Pal-SEQ ID NO: 23(SF)-NH₂, with an N-terminus Pal group, showed good ability to penetrate into the cell interior.

### Example 7. Conformational studies by circular dichroism

Circular dichroism spectra were recorded in aqueous solution and in a 60:40 H₂O:trifluoroethanol (TFE) mixture. The concentration used was 10 µM and the temperature (T) was adjusted to 5°C. Measurements were made between 190 and 250 nm using a 0.1 cm optical pitch cell. The experiments were carried out at a scanning rate of 50 nm/min, a response time of 2 s and a bandwidth of 1 nm. The spectra obtained are the average of 4 sweeps to which the baseline was corrected.

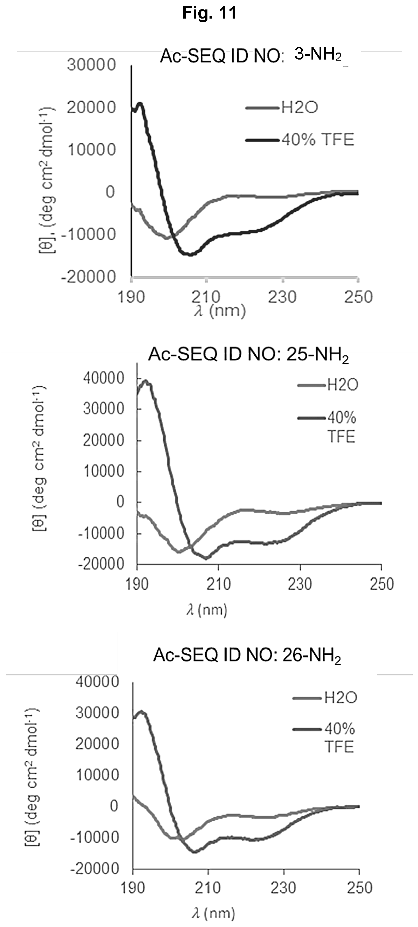

To calculate helicity, the equation *[θ]₂₂₂*/*[θ]_{max.}* was used, where *[θ]ₘₐₓ = (-44000+250T) (1-*/*r*/*n)*. As a finite length correction value (*k*), the value of *k =* 4 was taken, while *n* refers to the number of residues in the peptides. The helicity values of the peptides studied are given in Table 6, while the circular dichroism spectra are shown in Figure 11.

**Table 6. Helicity observed by circular dichroism for the peptides studied.**

| **Peptide** | **Helicity in H₂O (%)** | **Helicity in 60:40 H₂O/TFE (%)** |
|---|---|---|
| Ac-SEQ ID NO: 3-NH₂ | 2.7 | 32.3 |
| Ac-SEQ ID NO: 25-NH₂ | 10.7 | 46.2 |
| Ac-SEQ ID NO: 26-NH₂ | 10.8 | 35.5 |

From the values in table 6 and the graphs of Fig. 11, it can be deduced that the peptides studied show helicity between 2 and 10% in aqueous solution, which increase significantly in the presence of a helicogenic solvent such as TFE. The increase in helicity in H₂O when comparing the peptide Ac-SEQ ID NO: 25-NH₂ with Ac-SEQ ID NO: 3-NH₂, of 8%, shows that the incorporation of a K residue at the C-terminus induces a significant increase in the α-helix structuring of the peptide. The incorporation of an R residue after K in the peptide Ac-SEQ ID NO: 26-NH₂ has no significant effect on helicity with respect to Ac-SEQ ID NO: 25-NH₂. The data on circular dichroism in aqueous solution and H₂O:TFE mixture reliably demonstrate that these peptides tend to be structured in α-helix conformations, favoured by the incorporation of basic residues at the C-terminus.

### Example 8. Stability study in blood serum

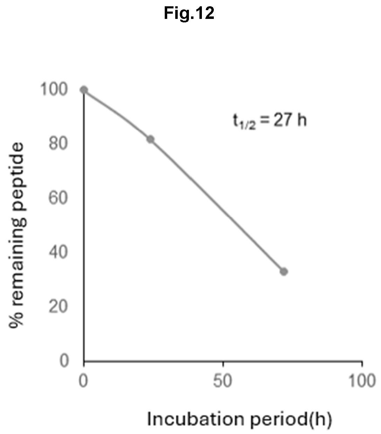

The stability study of the peptide Ac-SEQ ID NO: 26-NH₂ in human blood serum is shown in Figure 12. A 1 mM dilution of the peptide in water was prepared and 0.5 ml was taken and mixed with 0.5 ml of human serum. The resulting solution was incubated for 72 hours at 37°C. After 24 hours and 72 hours, 120 µl aliquots were taken and treated with 20 µl trifluoroacetic acid to precipitate the proteins. After 30 minutes at 4°C, the mixture was centrifuged at 13000 g and 4°C. The supernatant, once filtered, was analysed by UPLC-MS using a C18 Bridge column (3.0 mm x 50 mm, 2.5 µm) and a gradient of 15 and 50% CH₃CN (0.1% formic acid) in H₂O (0.1% formic acid) over 5 minutes.

As can be seen in Figure 12, 82% of the peptide Ac-SEQ ID NO: 26-NH₂ remained unchanged after 24 hours of incubation. Taking into account that the serum used was at a concentration of 50% and the percentages of peptide unchanged at different times, the half-life of Ac-SEQ ID NO: 26-NH₂ was estimated at 27 hours. This serum stability value is very suitable for potential application in the above indications.

**Example 9. Effects of Pal-SEQ ID NO: 23-NH₂ and Pal-SEQ ID NO: 25-NH₂ on I_{Na}.** To analyse the effect of S Pal-SEQ ID NO: 23-NH₂ and Pal-SEQ ID NO: 25-NH₂ on I_{Na} generated by human cardiac Nav1.5 channels, a protocol similar to that described in example 2.1 was used. CHO cells were transfected with the cDNA that encodes human Nav1.5 channels (SEQ ID NO: 21) and I_{Na} was recorded by the patch-clamp technique in its whole-cell configuration using the external and internal solutions the composition of which is described in example 2.1.

Figure 13 shows in panel A the I_{Na} -voltage density curves recorded in CHO cells expressing Nav1.5 channels that were incubated (white symbols) or not (black symbols) for 24 hours with the peptide described in Pal-SEQ ID NO: 23-NH₂ (called D9 in the figure) at a concentration of 10 µM. As can be observed, the peptide described in Pal-SEQ ID NO: 23-NH₂ was able to statistically significantly increase I_{Na} at various membrane potentials. Each point represents the mean±SEM of "n" experiments obtained from at least 3 different CHO cell plates. One-way ANOVA followed by the Tukey test. * P<0.05 vs. untreated control cells. Panel B of Figure 13 shows the normalised density of the maximum peak of I_{Na} recorded in CHO cells expressing Nav1.5 channels that had been treated (white squares) or not (black circles) for 24 hours with the peptide SEQ ID NO: 25 at a concentration of 10 µM. The white circles represent the normalised density of the maximum peak of I_{Na} recorded in CHO cells expressing Nav1.5 channels together with the cDNA that encodes the fragment containing amino acids 15-25 described in SEQ ID NO: 3 (SEQ ID NO: 5). As can be seen, the increase produced by the peptide Pal-SEQ ID NO: 23-NH₂ is similar to that produced by the cDNA that encodes the fragment containing amino acids 15-25 described in SEQ ID NO: 3 (SEQ ID NO: 5). In this figure, each point represents one experiment and the bars represent the mean±SEM of "n" experiments obtained from at least 3 different CHO cell plates.

Figure 14 shows in panel A the I_{Na} -voltage density curves recorded in CHO cells expressing Nav1.5 channels that were incubated (white symbols) or not (black symbols) for 24 hours with the peptide described in Pal-SEQ ID NO: 25-NH₂ (called D10 in the figure) at a concentration of 10 µM. As can be observed, the peptide Pal-SEQ ID NO: 25-NH2 was able to statistically significantly increase I_{Na} at various membrane potentials. Each point represents the mean±SEM of "n" experiments obtained from at least 3 different CHO cell plates. One-way ANOVA followed by the Tukey test. * P<0.05 vs. untreated control cells.

Panel B of Figure 14 shows the normalised density of the maximum peak of I_{Na} recorded in CHO cells expressing Nav1.5 channels that had been treated (white squares) or not (black circles) for 24 hours with the peptide SEQ ID NO: 29, at a concentration of 10 µM. The white circles represent the normalised density of the maximum peak of I_{Na} recorded in CHO cells expressing Nav1.5 channels together with the cDNA that encodes the fragment containing amino acids 15-25 described in SEQ ID NO: 3 (i.e., SEQ ID NO: 5). As can be seen, the increase produced by the peptide Pal-SEQ ID NO: 25-NH₂ is similar to that produced by the cDNA that encodes the fragment containing amino acids 15-25 described in SEQ ID NO: 3 (i.e., SEQ ID NO: 5). In this figure, each point represents one experiment and the bars represent the mean±SEM of "n" experiments obtained from at least 3 different CHO cell plates.

## Claims

1. Peptides of general formula R¹-X₁X₂RESLAX₃X₄X₅-R² (R¹-SEQ ID NO: 2-R²), in which
R1 is selected from hydrogen (H), arginine (R), acetyl (Ac), palmitoyl (Pal), Ac-R or Pal-R
X₁ is selected from F, Y or W,
X₂ is selected from T, S, C or M,
X₃ is selected from A, G, V, L or I,
X₄ is selected from I, G, A, V or L,
X₅ is selected from E or D, and
R2 is selected from OH, NH₂, K-NH₂, K(SF)-NH₂, or KR-NH₂,
where SF indicates fluorescent probe.

2. The peptide according to claim 1, wherein R1 is selected from H, Ac or Pal.

3. The peptide according to claim 2, wherein R2 is selected from alkyl OH, NH₂, K-NH₂, K(SF)-NH₂, or KR-NH₂.

4. The peptide according to any of claims 1-3, which is selected from the group consisting of SEQ ID NO: 3, Pal-SEQ ID NO: 23-NH₂ and/or Pal-SEQ ID NO: 25-NH₂.

5. The peptide according to any of claims 1-4, for use as a medicine.

6. The peptide according to claim 5, for use as a medicine in cardiac diseases.

7. The peptide according to claim 6, wherein the cardiac diseases belong to the group consisting of: ventricular arrhythmias associated with cardiac hypertrophy, cardiac insufficiency and/or hereditary syndromes secondary to mutations in the *SCN5A* or *KCNJ2* genes resulting in the loss of function in the Nav1.5 or Kir2.1 channels.

8. The peptide according to claim 7, wherein the hereditary syndromes belong to the group consisting of: Brugada Syndrome, Progressive cardiac conduction defect syndrome and Andersen-Tawil syndrome.

9. A cDNA molecule encoding the peptide described in SEQ ID NO: 3, Pal-SEQ ID NO: 25-NH₂ or Pal-SEQ ID NO: 25-NH₂.

10. The cDNA molecule according to claim 9, consisting of SEQ ID NO: 5, SEQ ID NO: 27 or SEQ ID NO: 28.

11. The cDNA molecules according to any of claims 9-10, for use as a medicine.

12. The cDNA molecules according to claim 11, for use as a medicine in cardiac diseases.

13. The cDNA molecules according to claim 12, wherein the cardiac diseases belong to the group consisting of: ventricular arrhythmias associated with cardiac hypertrophy, cardiac insufficiency and/or hereditary syndromes secondary to mutations in the *SCN5A* or *KCNJ2* genes resulting in the loss of function in the Nav1.5 or Kir2.1 channels.

14. The cDNA molecules according to claim 13, wherein the hereditary syndromes belong to the group consisting of: Brugada Syndrome, Progressive cardiac conduction defect syndrome and Andersen-Tawil syndrome.

15. A vector including a cDNA molecule as defined in any of claims 9-10.

16. A eukaryotic or prokaryotic cell containing any of the peptides, cDNA molecules or vectors as defined in claims 1-4, 9-10, 15.

17. A pharmaceutical composition comprising a peptide as defined in any of claims 1-4 and a pharmaceutically acceptable excipient.

18. A pharmaceutical composition comprising the molecule of any of claims 9-10 and a pharmaceutically acceptable excipient.

19. A pharmaceutical composition comprising the vector and/or the cell of any of claims 15-16 and a pharmaceutically acceptable excipient.

20. The pharmaceutical composition according to any of claims 17-19, for use in the prevention and/or treatment of cardiac diseases.

21. The pharmaceutical composition according to claim 20, wherein the cardiac diseases belong to the group consisting of: ventricular arrhythmias associated with cardiac hypertrophy, cardiac insufficiency and/or hereditary syndromes secondary to mutations in the *SCN5A* or *KCNJ2* genes resulting in the loss of function in the Nav1.5 or Kir2.1 channels.

22. The pharmaceutical composition according to claim 21, wherein the hereditary syndromes belong to the group consisting of: Brugada Syndrome, Progressive cardiac conduction defect syndrome and Andersen-Tawil syndrome.
